# EUROPÄISCHE PATENTSCHRIFT

(11) **EP 0 545 928 B1**
(45) Veröffentlichungstag und Bekanntmachung des Hinweises auf die Patenterteilung: **07.12.1994**
(21) Anmeldenummer: 91908226.3
(22) Anmeldetag: 29.04.1991
(51) Int. Cl.: A61M 25/00

(54) **SONDE ZUM EINFÜHREN IN EINE MENSCHLICHE KÖRPERHÖHLE**
PROBE DESIGNED TO BE INSERTED IN CAVITIES IN THE HUMAN BODY
SONDE S'INTRODUISANT DANS UNE CAVITE DU CORPS HUMAIN

(30) Priorität: 29.08.1990 DE 9012370 U
(43) Veröffentlichungstag der Anmeldung: 16.06.1993
(73) Patentinhaber: Moser, Franz, D-81476 München (DE)
(72) Erfinder: Billino, Helmut, D-81925 München (DE)
(74) Vertreter: von Hellfeld, Axel, Dr. Dipl.-Phys.
(86) Internationale Anmeldenummer: EP9100815
(87) Internationale Veröffentlichungsnummer: WO9204071

(56) Entgegenhaltungen:
- EP-A- 0 067 140
- WO-A-85/00526
- FR-A- 2 240 026
- GB-A- 189 127
- US-A- 1 045 326

## Beschreibung

Die Erfindung betrifft eine Sonde zum Einführen in eine menschliche Körperhöhle mit den Merkmalen des Oberbegriffs des Patentanspruches 1. Eine solche Sonde ist aus der EP-A-0 067 140 bekannt. Dort sind Schlitze nur am distalen Ende des Schlauches vorgesehen. Die Schlitze führen am Boden in Hohlräume, deren Durchmesser größer ist als die Breite des Schlitzes. Diese Hohlräume sind zusätzlich zum eigentlichen Hohlraum des Schlauches vorgesehen und über Verbindungslöcher mit diesem verbunden.

Die Schläuche solcher medizinischer Sonden bestehen zumeist aus flexiblem Kunststoff, wie z.B. PVC oder auch EVA. Die Materialwahl wird von den Anforderungen an diese Schläuche bestimmt. Zum einen sollen sie beim Einführen in den Körper eines Patienten hinreichend steif sein, um dabei auftretende Widerstände überwinden zu können, und zum anderen sollen sie im eingeführten Zustand weich und flexibel sein, um dem Patienten keine Beschwerden zu verursachen. Weiterhin muß das Material sterilisierbar und nicht toxisch sein und soll außerdem eine möglichst preiswerte Herstellung der Schläuche zulassen.

Alle Materialien, die diese Anforderungen erfüllen, können auch für den Schlauch der erfindungsgemäßen Sonde eingesetzt werden.

Der Begriff "Sonde" umfaßt ausdrücklich Katheter für medizinische Zwecke, insbesondere Absaugkatheter für die Atemwege, die Lunge und den Magen-Darmtrakt, sowie Katheter für die künstliche Ernährung, z.B. bei der perkutanen Gastrostomie.

In der Praxis werden solche Sonden häufig dazu benutzt, Flüssigkeiten wie etwa Schleim und andere Körpersekrete aus der Körperhöhle abzusaugen. Hierzu ist die Sonde an ihrem proximalen Ende mit einem auch Konnektor genannten Ansatz versehen, der zum einen die Verbindung der Sonde mit einer Unterdruckquelle, z.B. einer Saugpumpe, ermöglicht und zum anderen eine Steuerung des in der Sonde wirkenden Saugdrucks gestattet, z.B. durch mehr oder weniger starkes Verschließen einer seitlich am Ansatz ausgebildeten Öffnung mit einem Finger.

Häufig weisen die Sonden eine endständige Hauptöffnung auf. Es sind aber auch Sonden ohne diese endständige Hauptöffnung, also nur mit Öffnungen in der Mantelfläche, bekannt.

Beim Absaugen tritt das Problem auf, daß bei einer Okklusion der vorhandenen Öffnungen, z.B. durch Schleimpfropfen, das empfindliche Schleimhautgewebe etwa in den Atemwegen, der Lunge oder dem Magen und Darm, in die Öffnung oder die Öffnungen gesaugt und dadurch lokal beschädigt wird. Dem Patienten werden so zusätzliche Beschwerden verursacht, daneben ist auch die Gefahr einer Infektion beträchtlich erhöht.

Aus dem Stand der Technik sind unterschiedliche Sonden bekannt, mit denen versucht wird, dieses Problem zu lösen und eine gewebeschonendere Absaugung von Schleim und anderen Körpersekreten zu ermöglichen.

Es wurde z.B. vorgeschlagen, am distalen Ende des Schlauches ein Absaugkörbchen aus weicherem Material anzuordnen. Neben einer endständigen Öffnung im Absaugkörbchen sind außen in Längsvertiefungen mehrere Ausnehmungen angeordnet, durch die Luft in den Schlauch eintreten kann. Damit soll verhindert werden, daß die Sonde sich am umgebenden Gewebe festsaugt. Bei dieser Lösung besteht die Gefahr, daß das mit dem Schlauch verklebte Absaugkörbchen sich vom Schlauch löst und z. B. in der Lunge zurückbleibt. Abgesehen davon ist diese Lösung fertigungsaufwendig und entsprechend teuer in der Herstellung.

Ein weiterer Lösungsversuch besteht darin, einen Wulst mit einem bezüglich des Schlauchdurchmessers größeren Durchmesser um die endständige Hauptöffnung am distalen Ende herum anzuordnen. In proximaler Richtung gesehen sind bei dieser Lösung kurz hinter dem Wulst mehrere Öffnungen in der Mantelfläche über den Umfang des Schlauchs verteilt angeordnet. Durch die Öffnungen soll Luft angesaugt werden, die über den Wulst strömt und eine Art Luftpolster bildet, so daß sich der Katheter nicht am umgebenden Gewebe festsaugen kann. Der die endständige Hauptöffnung umgebende Wulst erhöht jedoch die Verletzungsgefahr für den Patienten beim Ein- und Ausführen einer solchen Sonde. Zudem ist auch diese Lösung teuer in der Herstellung.

Ein weiterer Lösungsvorschlag beschreibt eine Sonde mit einem biegbaren Schlauch, an dessen distalem Ende zwei kurze, sich gegenüberliegende Längsrillen auf der äußeren Mantelfläche angeordnet sind. Bezogen auf die Längserstreckung dieser Längsrillen sind etwa in der Mitte jeweils zwei Öffnungen in den Längsrillen angeordnet. Der Durchmesser der Öffnungen ist wesentlich kleiner als der Durchmesser der endständigen Hauptöffnung des Schlauches. Das dem distalen Ende des Schlauches benachbarte Ende der Längsrille weist zu diesem etwa denselben Abstand auf, den die beiden Öffnungen einer Längsrille voneinander haben. Die in den Längsrillen vertieft angeordneten Öffnungen sollen ein Festsaugen der Sonde am umgebenden Körpergewebe verhindern. Die Herstellung des Schlauches für diese Sonde ist kostenintensiv, weshalb die Sonde, wie auch die beiden vorbeschriebenen Sonden, im Vergleich zu herkömmlichen Sonden etwa vier- bis sechsmal teurer sind.

Aus der EP-A 0 212 159 ist eine Sonde bekannt, bei der ein distaler Endabschnitt des Schlauches sich zum distalen Ende konisch verjüngt. Neben einer endständigen Öffnung weist der distale Endabschnitt zahlreiche, über den Umfang verteilte Öffnungen in der Mantelfläche des Schlauches auf. Diese Öffnungen sind jedoch nicht vertieft in einer Rille angeordnet. Auf einem Teil des distalen Endabschnitts erstrecken sich über den Umfang gleichmäßig verteilt mehrere Rippen in axialer Richtung des Schlauches. Die Rippen dienen der Verstärkung und Stabilisierung des die Öffnungen aufweisenden distalen Endabschnitts des Schlauches und sollen gleichzeitig verhindern, daß den Schlauch umgebendes Gewebe die Öffnungen in der Mantelfläche okkludiert. In proximaler Richtung verjüngen sich die Rippen und enden etwa dort, wo der sich konisch verjüngende distale Endabschnitt in den unverjüngten Teil des Schlauches übergeht. Auch dieser Schlauch ist aufwendig herzustellen und daher teuer.

Die WO 85/00526 beschreibt einen Drainageschlauch mit einem enganliegenden, dünnwandigen Überzugsschlauch, der webartig perforiert ist. Dieser Überzugsschlauch ist aufwendig, sowohl hinsichtlich der Herstellung als auch bei der Handhabung des Drainageschlauches. Er kann sich bei Gebrauch verschieben oder gar vom Grundschlauch lösen.

Der Erfindung liegt die Aufgabe zugrunde, eine Sonde zum Einführen in eine menschliche Körperhöhle derart weiterzubilden, daß ein gewebeschonendes Absaugen von Flüssigkeiten, z. B. Körpersekreten, ermöglicht ist, wobei der Herstellungspreis dieser Sonde nicht wesentlich über dem herkömmlicher Sonden liegt.

Eine diese Aufgabe erfindungsgemäß lösende Sonde ist im Patentanspruch 1 gekennzeichnet.

Vorteilhafte Ausgestaltungen der Sonde sind in den abhängigen Ansprüchen beschrieben.

Die biegbaren Schläuche für derartige Sonden werden durch Extrudieren hergestellt. Der Erfindung liegt die Erkenntnis zugrunde, daß beim Extrudieren ohne zusätzlichen Aufwand eine oder mehrere Rillen auf der äußeren Mantelfläche des Schlauches erzeugt werden können, wenn das Formwerkzeug entsprechend gestaltet ist. Der aus dem Extruder kommende Schlauch weist also uber seine gesamte Länge eine durch das Formwerkzeug vorgegebene Anzahl durchgehender und im wesentlichen in Extrudierrichtung verlaufender Rillen auf. Zur Anpassung an die verschiedenen Einsatzorte, wie z.B. Magen oder Lunge, wird der Schlauch in entsprechende Längen geschnitten.

Die Anbringung zumindest einer Öffnung in jeder Rille nahe dem distalen Ende des Schlauches erfolgt auf herkömmliche Weise. Die Größe der Öffnung oder der Öffnungen wird in Abhängigkeit ihrer Anzahl, des Durchmessers einer gegebenenfalls vorhandenen endständigen Hauptöffnung und des angelegten Saugdrucks so bemessen, daß einerseits nicht zuviel Luft während des Absaugens durch sie hindurchströmt, wodurch die Saugleistung an der endständigen Hauptöffnung, sofern vorhanden, verschlechtert wurde, und andererseits die Öffnung bzw. die Öffnungen nicht allzuleicht okkludiert werden. Soll die Sonde als Magensonde eingesetzt werden, so sind die Öffnungen im allgemeinen größer zu bemessen als bei einer zur bronchialen Sekretabsaugung vorgesehenen Sonde.

Die Form der öffnungen ist bevorzugt rund oder oval, hat jedoch keinen wesentlichen Einfluß auf die Funktion der Sonde, so daß die Öffnungen im Einzelfall auch andere, geeignet erscheinende Formen haben können.

Eine bevorzugte Ausfuhrungsform der erfindungsgemäßen Sonde weist mehrere durchgehende und sich uber die gesamte Länge des Schlauches erstreckende Rillen auf, in denen zumindest jeweils eine Öffnung nahe dem distalen Ende des Schlauches angeordnet ist. Die Anzahl der Öffnungen pro Rille wird dem Einsatzbereich der Sonde, d.h. dem sie umgebenden Körpergewebe und der Konsistenz der abzusaugenden Flussigkeit angepaßt. Bei dieser Ausfuhrungsform erstrecken sich die Rillen geradlinig in axialer Richtung des Schlauches.

Bei einer anderen Ausfuhrungsform sind eine oder mehrere Rillen wendelförmig oder wellenförmig auf der Mantelfläche des Schlauches ausgebildet. Wendelförmige Rillen lassen sich durch Drehen des gerade extrudierten Schlauches um seine axiale Achse erzielen, während sich wellenförmige Rillen durch abwechselndes Hin- und Herdrehen des gerade extrudierten Schlauches um seine axiale Achse erzeugen lassen. Es ist auch möglich, statt des Schlauches das Formwerkzeug während des Extrudierens in entsprechender Weise zu drehen.

Die Funktion der erfindungsgemäßen Sonde ist nicht auf eine bestimmte Rillenform oder auf eine bestimmte Form des Schlauches beschränkt, so daß die Querschnittsform der äußeren und der inneren Mantelfläche des Schlauches sowie der Rillen, die nur durch das Formwerkzeug vorgegeben wird, in weiten Grenzen dem jeweiligen Einsatzzweck oder auch neuesten Erkenntnissen angepaßt werden kann. So kann z.B. die Querschnittsform der äußeren Mantelfläche kreisrund oder kleeblattförmig, aber auch oval oder vieleckig sein. Der Querschnitt der inneren Mantelfläche kann der äußeren Form angepaßt sein, wird jedoch bevorzugt rund oder zumindest oval sein. Der Querschnitt der Rillen kann halbkreisförmig, halbellipsenförmig, rechteckig, U-förmig oder auch dreieckig ausgefuhrt sein.

Normalerweise entspricht der Querschnittsdurchmesser der Rille im wesentlichen dem Durchmesser der Öffnung in der Rille. Je nach Anwendungsfall kann es jedoch vorteilhaft sein, entweder den Durchmesser der Öffnung bzw. der Öffnungen kleiner zu wählen als die größte Rillenbreite oder aber auch den Durchmesser der Öffnung(en) größer als die größte Rillenbreite zu wählen.

Um Verletzungen des Patienten während des Einfuhrens der Sonde zu vermeiden, ist das distale Ende (Einfuhrende) bevorzugt abgerundet.

Die Erfindung wird im folgenden anhand bevorzugter Ausfuhrungsbeispiele näher erläutert. Es zeigt:
- Fig. 1: einen Schlauch einer ersten Ausfuhrungsform der erfindungsgemäßen Sonde in perspektivischer Darstellung,
- Fig. 2: einen Querschnitt entlang der Linie II-II in Fig. 1, und
- Fig. 3: den Schlauch einer zweiten Ausfuhrungsform in perspektivischer Darstellung.

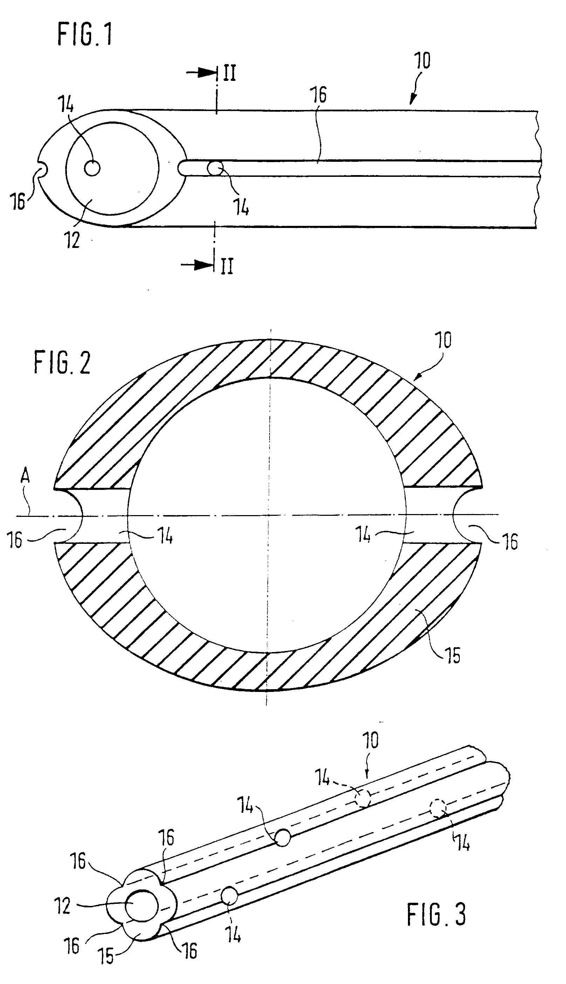

In Fig. 1 ist ein distaler Endabschnitt eines flexiblen Schlauches 10 gezeigt, der Teil einer in eine menschliche Körperhöhle einfuhrbaren Sonde ist. An seinem distalen Ende weist der Schlauch 10 eine kreisrunde endständige Hauptöffnung 12 auf.

Auf der äußeren Mantelfläche des Schlauches 10 erstrecken sich zwei durchgehende und einander gegenuberliegende gerade Rillen 16. Nahe dem distalen Ende des Schlauches 10 ist in den Rillen 16 jeweils eine Öffnung 14 angeordnet, die sich durch die Schlauchwandung 15 erstreckt. Die Öffnungen 14 liegen einander gegenuber und weisen einen kreisförmigen Querschnitt auf.

Aus Fig. 2 ist ersichtlich, daß der Querschnitt der äußeren Mantelfläche des Schlauches 10 oval ist, während der Querschnitt der inneren Mantelfläche kreisringförmig ist. Die ovale äußere Gestalt des Schlauches 10 erleichtert das Ein- und Ausfuhren der Sonde. Durch den ovalen Querschnitt der äußeren Mantelfläche ändert sich die Wanddicke in Umfangsrichtung des Schlauches 10.

Die beiden Rillen 16 sind einander gegenuberliegend im Bereich der größten Wanddicke und bezuglich ihres Querschnitts symmetrisch zu einer Symmetrieachse A angeordnet, die den ovalen Querschnitt der äußeren Mantelfläche in zwei Längshälften teilt und gleichzeitig Symmetrieachse der Öffnungen 14 ist.

Der Querschnitt der Rillen 16 ist halbkreisförmig, wobei der Durchmesser der Rillen 16 dem Durchmesser der Öffnungen 14 entspricht. Der Durchmesser der Öffnungen 14 ist beträchlich kleiner als der Durchmesser der endständigen Hauptöffnung 12.

In Fig. 3 ist der distale Endabschnitt des flexiblen Schlauches 10 einer zweiten Ausfuhrungsform der Sonde gezeigt. An seinem distalen Ende weist der Schlauch 10 die kreisrunde endständige Hauptöffnung 12 auf. Auf der äußeren Mantelfläche des Schlauches 10 erstrecken sich vier durchgehende gerade Rillen 16, die in Umfangsrichtung gesehen jeweils gleichen Abstand voneinander aufweisen. Bei dieser Ausfuhrungsform entstehen die Rillen 16 bereits durch die Formgebung der äußeren Mantelfläche, die eine Querschnittsform ähnlich der Form eines vierblättrigen Kleeblattes aufweist.

Wie aus Fig. 3 ersichtlich, ist jeweils eine sich durch die Schlauchwandung 15 erstreckende Öffnung 14 in jeder Rille 16 angeordnet. Die Öffnungen 14 weisen einen Durchmesser von etwa 2,5 mm auf und sind in axialer Richtung des Schlauches in einem Abstand von etwa 2 cm wendelförmig auf der Mantelfläche des Schlauches 10 angeordnet. Die dem distalen Ende des Schlauches 10 benachbarte Öffnung 14 weist zu diesem ebenfalls einen Abstand von etwa 2 cm auf.

Der Querschnitt der Rillen 16 ist bedingt durch die Querschnittsform der äußeren Mantelfläche angenähert herzförmig. Auch mit dieser zweiten Ausfuhrungsform der Sonde wird erreicht, daß empfindliches Gewebe in einer Körperhöhle die Öffnungen 14 nicht vollständig verschließen kann. Dadurch ist verhindert, daß beim Betrieb der Sonde das Gewebe durch in ihr herrschendem Unterdruck in die Öffnungen hineingesaugt und geschädigt wird.

## Patentansprüche

1. Sonde zum Einführen in eine menschliche Körperhöhle mit einem biegbaren, einen inneren Hohlraum aufweisenden Schlauch (10) und auf seiner äußeren Mantelfläche vertieft angeordneten Öffnungen (14), insbesondere nahe dem distalen Ende der Sonde, wobei die Öffnungen (14) im Boden zumindest einer Rille (16) angeordnet sind,
dadurch **gekennzeichnet,** daß die Rille (16) sich auf der Mantelfläche über die gesamte Länge des Schlauches (10) erstreckt und daß die Öffnungen (14) vom Boden der Rille (16) zum inneren Hohlraum des Schlauches (10) führen.

2. Sonde nach Anspruch 1,
dadurch **gekennzeichnet,** daß der biegbare Schlauch (10) an seinem distalen Ende eine endständige Hauptöffnung (12) aufweist.

3. Sonde nach Anspruch 1 oder 2,
dadurch **gekennzeichnet,** daß in mehreren sich über die gesamte Länge des Schlauches (10) erstreckenden Rillen (16) zumindest jeweils eine Öffnung (14) angeordnet ist.

4. Sonde nach einem der Ansprüche 1 bis 3,
dadurch **gekennzeichnet,** daß die Rille (16) sich geradlinig über die gesamte Länge des Schlauches (10) erstreckt.

5. Sonde nach einem der Ansprüche 1 bis 3,
dadurch **gekennzeichnet,** daß die Rille (16) wendelförmig oder wellenförmig auf der äußeren Mantelfläche ausgeformt ist.

6. Sonde nach einem der vorhergehenden Ansprüche,
dadurch **gekennzeichnet,** daß der Querschnitt der Rille (16) halbkreis-, halbellipsen-, U-förmig, rechteckig oder dreieckig ist.

7. Sonde nach einem der vorhergehenden Ansprüche,
dadurch **gekennzeichnet,** daß der Querschnittsdurchmesser der Rille (16) im wesentlichen dem Durchmesser der Öffnung (14) entspricht.

8. Sonde nach einem der Ansprüche 1 bis 6,
dadurch **gekennzeichnet,** daß die größte Rillenbreite größer ist als der Durchmesser der Öffnung (14).

9. Sonde nach einem der Ansprüche 1 bis 6,
dadurch **gekennzeichnet,** daß der Durchmesser der Öffnung (14) größer ist als die größte Rillenbreite.

## Claims

1. A probe for introduction into a human body cavity, comprising a flexible hose (10) with an internal hollow space and with apertures (14) which are located sunk in the outside surface of the hose, especially near the distal end of the probe, the apertures (14) being disposed in the bottom of at least one groove (16), **characterized** in that the groove (16) extends throughout the length of the hose (10) in the outside surface thereof, and that the apertures (14) lead from the bottom of the groove (16) to the internal hollow space of the hose (10).

2. The probe as claimed in claim 1, characterized in that the flexible hose (10) has a terminal main opening (12) at its distal end.

3. The probe as claimed in claim 1 or 2, characterized in that there is at least one aperture (14) each in a plurality of grooves (16) extending throughout the length of the hose (10).

4. The probe as claimed in any one of claims 1 to 3, characterized in that the groove (16) is rectilinear for the whole length of the hose (10).

5. The probe as claimed in any one of claims 1 to 3, characterized in that the groove (16) is formed in helical or sinusoidal configuration in the outside surface.

6. The probe as claimed in any one of the preceding claims, characterized in that the cross section of the groove (16) is semicircular, semi-elliptical, U-shaped, rectangular, or triangular.

7. The probe as claimed in any one of the preceding claims, characterized in that the cross sectional diameter of the groove (16) essentially corresponds to the diameter of the aperture (14).

8. The probe as claimed in any one of claims 1 to 6, characterized in that the greatest groove width is greater than the diameter of the aperture (14).

9. The probe as claimed in any one of claims 1 to 6, characterized in that the diameter of the aperture (14) is greater than the greatest groove width.

## Revendications

1. Sonde à introduire dans une cavité corporelle humaine, comportant un tube flexible (10) présentant une cavité interne et des ouvertures (14) disposées en creux sur son enveloppe externe, notamment à proximité de l'extrémité distale de la sonde, les ouvertures (14) étant ménagées dans le fond d'au moins une rainure (16), caractérisée en ce que la rainure (16) s'étend sur l'enveloppe sur toute la longueur du tube (10) et en ce que les ouvertures (14) mènent du fond de la rainure (16) à la cavité interne du tube (10).

2. Sonde selon la revendication 1, caractérisée en ce que le tube flexible (10) présente à son extrémité distale une ouverture principale terminale(12).

3. Sonde selon la revendication 1 ou 2, caractérisée en ce qu'au moins une ouverture (14) est ménagée dans respectivement plusieurs rainures (16) s'étendant sur toute la longueur du tube (10).

4. Sonde selon une des revendications 1 à 3, caractérisée en ce que la rainure (16) s'étend linéairement sur toute la longueur du tube (10).

5. Sonde selon une des revendications 1 à 3, caractérisée en ce que la rainure (16) est de forme hélicoïdale ou sinusoïdale sur l'enveloppe externe.

6. Sonde selon une des revendications précédentes, caractérisée en ce que la section transversale de la rainure (16) est hémisphérique, hémiellipsoïdale, en U, rectangulaire ou triangulaire.

7. Sonde selon une des revendications précédentes, caractérisée en ce que le diamètre de la section transversale de la rainure (16) correspond sensiblement au diamètre de l'ouverture (14).

8. Sonde selon une des revendications 1 à 6, caractérisée en ce que la plus grande largeur de la rainure est supérieure au diamètre de l'ouverture (14).

9. Sonde selon une des revendications 1 à 6, caractérisée en ce que le diamètre de l'ouverture (14) est supérieur à la plus grande largeur de la rainure.
